(19) Europäisches Patentamt
European Patent Office
Office européen des brevets

(11) Veröffentlichungsnummer: **0 421 224 B1**

(12) # EUROPÄISCHE PATENTSCHRIFT

(45) Veröffentlichungstag der Patentschrift: **24.08.94**

(51) Int. Cl.$^5$: **C07D 261/18**, C07D 413/04

(21) Anmeldenummer: **90118273.3**

(22) Anmeldetag: **24.09.90**

(54) **Verfahren zur Herstellung von Isoxazol-5-carbonsäureamid-Derivaten.**

(30) Priorität: **03.10.89 DE 3932917**

(43) Veröffentlichungstag der Anmeldung:
**10.04.91 Patentblatt 91/15**

(45) Bekanntmachung des Hinweises auf die Patenterteilung:
**24.08.94 Patentblatt 94/34**

(84) Benannte Vertragsstaaten:
**BE CH DE ES FR GB IT LI NL**

(56) Entgegenhaltungen:
**EP-A- 0 337 263**

**JOURNAL OF HETEROCYCLIC CHEMISTRY, Band 22, Nr. 6, November-Dezember 1985,Seiten 1561-1565, Provo, Utah, US; A. CAMPARINI et al.: "Synthesis andphotochemical reactivity of 3-methylisoxazolo[4,5-d]pyridazines [1]"**

**JOURNAL OF THE CHEMICAL SOCIETY, PERKIN TRANSACTIONS I, 1987, Seiten 1005-1009;R. NESI et al.: "A new spiro annelation reaction in the isoxazole series:Applications and limits. Part 2. Reactivity of ethyl 4-nitro-3-phenylisoxazole-5-carboxilate with nitrogenbinucleophiles**

(73) Patentinhaber: **BASF Aktiengesellschaft
Carl-Bosch-Strasse 38
D-67063 Ludwigshafen (DE)**

(72) Erfinder: **Kuekenhoehner, Thomas, Dr.
Seidelstrasse 2
W-6710 Frankenthal (DE)**
Erfinder: **Freund, Wolfgang, Dr.
Johann-Gottlieb-Fichte-Strasse 71
W-6730 Neustadt (DE)**
Erfinder: **Maywald, Volker, Dr.
Berner Weg 24
W-6700 Ludwigshafen (DE)**
Erfinder: **Goetz, Norbert, Dr.
Schoefferstrasse 25
W-6520 Worms 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft ein Verfahren zur Herstellung von Isoxazol-5-carbonsäureamid-Derivaten der allgemeinen Formel I

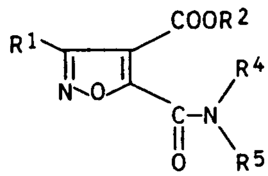

I

in der

$R^1$ ein aliphatischer Rest mit 1 bis 20, ein cycloaliphatischer Rest mit 3 bis 10, ein aromatischer Rest mit 6 bis 10, ein heteroaromatischer oder heterocyclischer Rest mit 3 bis 10 oder ein araliphatischer Rest mit 4 bis 12 Kohlenstoffatomen ist, wobei diese Reste mit unter den Reaktionsbedingungen inerten Substituenten substituiert sein können,

$R^2$ eine Alkylgruppe ist,

und in der die Reste

$R^4$ und $R^5$ gleich oder verschieden sind und für Wasserstoff, $C_1$- bis $C_6$-Alkyl-, $C_1$- bis $C_6$-Cyanoalkyl-, $C_2$- bis $C_6$-Alkenyl-, $C_2$- bis $C_6$-Alkinyl- und/oder für mit 1 bis 3 $C_1$- bis $C_4$-Alkylsubstituenten substituierte oder unsubstituierte $C_3$-bis $C_8$-Cycloalkylgruppen stehen oder zu einem 5- bis 8-gliedrigen, cycloaliphatischen Ring, der ein weiteres Stickstoff- oder ein Sauerstoffatom enthalten kann, verbunden sind,

mit der Maßgabe, daß falls $R^1$ die Methylgruppe und $R^4$ und $R^5$ eine Kombination aus Wasserstoff und der Isopropylgruppe darstellen, $R^2$ keine Ethylgruppe ist.

Isoxazol-5-carbonsäureamid-Derivate der allgemeinen Formel I sind wichtige Zwischenprodukte zur Herstellung von Herbiziden, beispielsweise den in der EP-A-337 263 beschriebenen.

Der vorliegenden Erfindung lag deshalb die Aufgabe zugrunde, ein möglichst einfaches und wirtschaftliches Verfahren zu finden, daß es erlaubt, die über die 1,3-dipolare Cycloaddition von Acetylendicarbonsäure-Diestern mit entsprechend substituierten, in situ erzeugten Nitriloxiden gut zugänglichen (vgl. z.B. Chem. Pharm. Bull. 28, 3296-3303 (1980); Tetrahedron 30, 1365-1371 (1974)) Isoxazol-4,5-dicarbonsäure-Diester der allgemeinen Formel II möglichst selektiv in die Isoxazol-5-carbonsäureamid-Derivate I umzuwandeln.

Dementsprechend wurde ein Verfahren zur Herstellung von Isoxazol-5-carbonsäureamid-Derivaten der allgemeinen Formel I

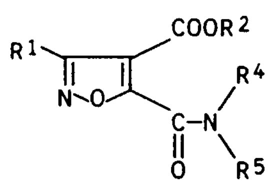

I

in der

$R^1$ ein aliphatischer Rest mit 1 bis 20, ein cycloaliphatischer Rest mit 3 bis 10, ein aromatischer Rest mit 6 bis 10, ein heteroaromatischer oder heterocyclischer Rest mit 3 bis 10 oder ein araliphatischer Rest mit 4 bis 12 Kohlenstoffatomen ist, wobei diese Reste mit unter den Reaktionsbedingungen inerten Substituenten substituiert sein können,

$R^2$ eine Alkylgruppe ist,

und in der die Reste

$R^4$ und $R^5$ gleich oder verschieden sind und für Wasserstoff, $C_1$- bis $C_6$-Alkyl-, $C_1$- bis $C_6$-Cyanoalkyl-, $C_2$- bis $C_6$-Alkenyl-, $C_2$- bis $C_6$-Alkinyl- und/oder für mit 1 bis 3 $C_1$- bis $C_4$-Alkylsubstituenten substituierte oder unsubstituierte $C_3$- bis $C_8$-Cycloalkylgruppen stehen oder zu einem 5- bis 8-gliedrigen, cycloaliphatischen Ring, der ein weiteres Stickstoff- oder ein Sauerstoffatom enthalten kann, verbunden sind,

mit der Maßgabe, daß falls $R^1$ die Methylgruppe und $R^4$ und $R^5$ eine Kombination aus Wasserstoff und der

Isopropylgruppe darstellen, $R^2$ keine Ethylgruppe ist, gefunden, das dadurch gekennzeichnet ist, daß man Isoxazol-4,5-dicarbonsäure-diester der allgemeinen Formel II

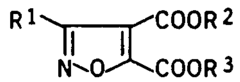

II

in der

$R^3$ für eine Alkylgruppe steht, die gleich oder verschieden von der Alkylgruppe $R^2$ ist, mit einem Amin der allgemeinen Formel III

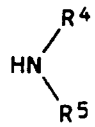

III

bei Temperaturen von 0 bis 100 °C umsetzt, wobei das Amin in mehr als äquimolaren Mengen, bezogen auf II, verwendet wird.

überraschenderweise führt die erfindungsgemäße Umsetzung der Isoxazol-4,5-dicarbonsäure-Diester II mit den Aminen III praktisch ausschließlich zu den Isoxazol-5-carbonsäureamid-Derivaten I. Die entsprechenden Isoxazol-4-carbonsäureamid-Derivate IV

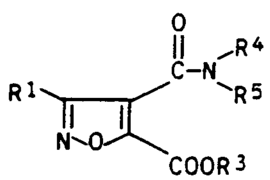

IV

und die Isoxazol-4,5-dicarbonsäure-diamide V

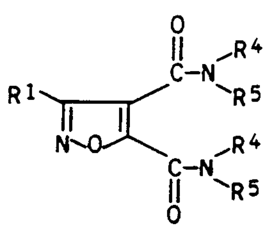

V

deren Bildung bei der erfindungsgemäßen Umsetzung in beträchtlichem Ausmaß zu erwarten war, werden unter den angewandten Reaktionsbedingungen nicht oder nur in vernachlässigbar geringen Ausmaß gebildet.

Im erfindungsgemäßen Verfahren wird der als Ausgangsverbindung benutzte Isoxazol-4,5-dicarbonsäure-diester II mit dem Amin III bei Temperaturen von 0 bis 100 °C, besonders bevorzugt bei 20 bis 80 °C, umgesetzt. Dabei wird zweckmäßigerweise die Umsetzung sterisch nicht gehinderter Amine bei Temperaturen von 20 bis 60 °C, durchgeführt, wohingegen bei Verwendung von Aminen III mit einem oder zwei sterisch anspruchsvollen Substituenten $R^4$ und $R^5$ im allgemeinen bei höheren Temperaturen, insbesondere bei 60 bis 80 °C, gearbeitet wird.

Die erfindungsgemäße Umsetzung läßt sich vorteilhaft in einem unter den Reaktionsbedingungen inerten Lösungsmittel durchführen. Vorzugsweise verwendete Lösungsmittel sind Ether, wie Diethylether, Diisopropylether, Dimethoxyethan, Methyl-tert. -Butylether, Tetrahydrofuran oder Dioxan, Halogenkohlenwasserstoffe, wie Methylenchlorid oder Chloroform, aromatische Kohlenwasserstoffe, wie Benzol, Toluol oder Xylole sowie Sulfoxide, wie Dimethylsulfoxid oder Sulfolan. Besonders bevorzugte Lösungsmittel sind jedoch Alkohole, wie Methanol, Ethanol, Propanol, Isopropanol, Butanol, tert.-Butanol u.s.w.. Es können

selbstverständlich auch Lösungsmittelgemische benutzt werden.

Erfindungsgemäß wird das zur Amidierung verwendete Amin im Überschuß bezüglich des Isoxazol-4,5-dicarbonsäure-diesters II verwendet. Bei einer besonders bevorzugten Arbeitsweise kann das überschüssige Amin selbst als Lösungsmittel fungieren.

Das Amin III kann in praktisch beliebigem, molarem Überschuß bezüglich des Reaktanten II eingesetzt werden. Vorteilhaft wird das Amin III bezüglich des Diesters II jedoch in der bis 5-fach molaren Menge angewandt.

Das erfindungsgemäße Verfahren wird im allgemeinen bei Atmosphärendruck ausgeführt. Je nach Art des verwendeten Amins oder Lösungsmittels, kann es sich jedoch besonders vorteilhaft auswirken, wenn die Umsetzung unter erhöhtem Druck, insbesondere unter autogen erhöhtem Druck in einem Autoklaven durchgeführt wird. Diese Maßnahme führt insbesondere dann zu vorteilhaften Ergebnissen, wenn niedrigsiedende Amine III, wie Methylamin, Ethylamin oder Isopropylamin oder niedrigsiedende Lösungsmittel, wie Diethylether oder Methanol, verwendet werden. Selbstverständlich kann das erfindungsgemäße Verfahren auch unter exogen erhöhtem Druck betrieben werden, zweckmäßigerweise im Druckbereich von 1 bis 50 bar, im allgemeinen werden durch diese Vorgehensweise aber keine weiteren Verbesserungen des Verfahrensergebnisses erzielt.

Das erfindungsgemäße Verfahren kann auf konventionelle Weise diskontinuierlich in Rührkesseln oder Rührautoklaven als auch kontinuierlich in Rührkesselkaskaden oder Rohrreaktoren betrieben werden. Insgesamt weist das erfindungsgemäße Verfahren keine verfahrenstechnischen Besonderheiten auf, so daß sich weitere Angaben hierzu erübrigen.

Je nach den angewandten Verfahrensbedingungen, wie Druck und Temperatur und je nach den verwendeten Ausgangsverbindungen, ist die Umsetzung im allgemeinen nach 1 bis 10 Stunden Reaktionsdauer beendet. Da die gewünschten Isoxazol-5-carbonsäureamid-Derivate im erfindungsgemäßen Verfahren mit einer hohen Selektivität gebildet werden, genügt es in vielen Fällen zur Aufarbeitung einfach das Losungsmittel und/oder das überschüssige Amin III abzudestillieren, um ein für die Weiterverarbeitung genügend reines Produkt I zu erhalten. Es ist aber auch möglich das Reaktionsgemisch auf herkömmliche Weise, Extraktion des überschüssigen Amins mit verdünnten, wäßrigen Säuren und Isolierung des Produkts I aus der organischen Phase, aufzuarbeiten. Diese Verfahrensweise wird insbesondere dann bevorzugt angewandt, wenn die abzutrennenden Amine relativ schwerflüchtige Substanzen sind.

Nach dem erfindungsgemäßen Verfahren können die Isoxazol-4,5-dicarbonsäure-Diester II mit praktisch allen primären bzw. sekundären Aminen der Formeln IIIa ($R^4$ = H) bzw. IIIb ($R^4 \neq$ H)

$R^5$—$NH_2$

IIIa

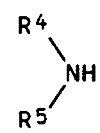

IIIb

selektiv zu den entsprechenden Isoxazol-5-carbonsäureamid-Derivaten I umgesetzt werden.

Vorzugsweise werden aber Amine III eingesetzt, in denen $R^4$ und $R^5$ gleich oder verschieden sind und für Wasserstoff, $C_1$- bis $C_6$-Alkyl, $C_1$- bis $C_6$-Cyanoalkyl-, $C_2$- bis $C_6$-Alkenyl-, $C_2$- bis $C_6$-Alkinyl- und/oder für mit 1 bis 3 $C_1$- bis $C_4$-Alkylsubstituenten substituierte oder unsubstituierte $C_3$- bis $C_8$-Cycloalkylgruppen stehen. Des weiteren werden vorzugsweise Amine III verwendet, in denen die Reste $R^4$ und $R^5$ miteinander zu einem 5-bis 8-gliedrigen, cycloaliphatischen Ring, der ein weiteres Stickstoff-oder ein Sauerstoffatom enthalten kann, verbunden sind.

Besonders bevorzugt werden die Isoxazol-4,5-dicarbonsäure-Diester II mit einfachen primären Aminen, wie Methylamin, Ethylamin, Propylamin, Isopropylamin, Isobutylamin, tert.-Butylamin, Neopentylamin, und 2-Amino-2-methylbutan,
ungesättigten Aminen, wie 3-Amino-3-methyl-but-1-in oder 3-Amino-3-methyl-but-1-en,
Cyano-substituierten Aminen, wie 2-Amino-2-cyano-propan, cyclischen primären Aminen, wie Cyclopropylamin, Cyclopentylamin, Cyclohexylamin, 1-Amino-1-methyl-cyclopropan,
1-Amino-2-methyl-cyclopropan, 1-Amino-1-methyl-cyclopentan, 1-Amino-1-methyl-cyclohexan, 1-Amino-1-ethyl-cyclopropan oder 1-Amino-1-ethyl-4-methyl-cyclohexan,
einfachen, sekundären Aminen, wie Dimethylamin, Diethylamin, Dipropylamin, Dibutylamin oder Diisopropylamin,
sowie mit cyclischen, sekundären Aminen, wie Pyrrolidin, Piperidin, Imidazolidin, Piperazin, Morpholin, 3-Methylmorpholin oder 3,5-Dimethylmorpholin
umgesetzt.

Nach dem erfindungsgemäßen Verfahren können Isoxazol-4,5-dicarbonsäure-diester der allgemeinen Formel II zu den entsprechenden Isoxazol-5-carbonsäure-Derivaten umgesetzt werden. So können Diester II, in denen der $R^1$ ein aliphatischer Rest mit 1 bis 20, ein cycloaliphatischer Rest mit 3 bis 10, ein aromatischer Rest mit 6 bis 10, ein heteroaromatischer oder heterocyclischer Rest mit 3 bis 10 oder ein araliphatischer Rest mit 4 bis 12 Kohlenstoffatomen ist, verwendet werden. Die Begrenzung der Anzahl der Kohlenstoffatome der Reste $R^1$ ist allein durch die Nützlichkeit und Verwendbarkeit der betreffenden Verbindungen begründet und beruht nicht auf einer mangelnden Durchführbarkeit des erfindungsgemäßen Verfahrens bei größeren Resten $R^1$.

Vorteilhaft können nach dem erfindungsgemäßen Verfahren Isoxazol-4,5-dicarbonsäure-diester umgesetzt werden, in denen der Rest $R^1$ eine $C_1$- bis $C_{10}$-, insbesondere eine $C_1$- bis $C_6$-Alkylgruppe, eine $C_2$- bis $C_{10}$-, insbesondere eine $C_2$- bis $C_6$-Alkenylgruppe, eine $C_3$- bis $C_8$-, insbesondere $C_3$- bis $C_7$-Cycloalkylgruppe, ein 5- bis 7-gliedriger, 1 oder 2 der Heteroatome Sauerstoff, Stickstoff und/oder Schwefel, insbesondere Sauerstoff und/oder Schwefel enthaltender, aromatischer oder aliphatischer Heterocyclus, oder eine Phenyl- oder Benzylgruppe ist.

Die Reste $R^1$ können weiterhin substituiert sein. Die Art und die Anzahl des Substituenten kann, selbstverständlich unter der Voraussetzung des chemisch möglichen, insoweit beliebig gewählt werden, als sich die Substituenten unter den Reaktionsbedingungen gegenüber dem Amin III chemisch inert verhalten.

So können die Alkylgruppen $R^1$, je nach ihrer Größe 1, 2, 3, 4 oder 5, vorzugsweise bis zu 3, gleiche oder verschiedene Substituenten, wie $C_3$-bis $C_7$-Cycloalkyl-, $C_1$- bis $C_3$-Alkoxy-, Cyano- oder Phenylgruppen tragen, wobei die Phenylsubstituenten wiederum mit bis zu 3, vorzugsweise mit einem oder 2 der Substituenten Halogen, $C_1$- bis $C_4$-Alkyl, $C_1$- bis $C_4$-Alkoxy, Cyano oder Nitro substituiert sein können und wobei das Substitutionsmuster dieser Phenylsubstituenten im allgemeinen nicht kritisch ist.

Die Alkylgruppen $R^1$ können geradkettig oder verzweigt sein. Besonders vorteilhaft können erfindungsgemäß Isoxazol-4,5-dicarbonsäure-diester umgesetzt werden, in denen $R^1$ eine Alkylgruppe ist, welche mit den Substituenten $C_1$- bis $C_4$-Alkoxy und/oder Phenyl substituiert sind, wobei der Phenylsubstituent vorzugsweise Halogen- und/oder $C_1$- bis $C_4$-Alkylsubstituenten trägt. Vorteilhaft lassen sich nach dem erfindungsgemäßen Verfahren auch Isoxazolcarbonsäureamide I herstellen, in denen der Rest $R^1$ Acetal- und/oder Ketal-Gruppierungen enthält.

Ist $R^1$ eine Cycloalkylgruppe, so kann diese je nach ihrer Größe mit 1, 2, 3, 4 oder 5, vorzugsweise mit bis zu 3, gleichen oder verschiedenen $C_1$-bis $C_4$-Alkyl- und/oder $C_1$- bis $C_3$-Alkoxysubstitutenten substituiert sein.

Nach dem erfindungsgemäßen Verfahren können vorteilhaft auch Isoxazol-5-carbonsäureamid-Derivate I produziert werden, in denen der Rest $R^1$ ein 5- bis 7-gliedriger Heterocyclus ist, der mit 1 bis 3 gleichen oder verschiedenen $C_1$- bis $C_4$-Alkyl- und/oder $C_1$- bis $C_4$-Alkoxygruppen substituiert sein kann. Dabei kann der Heterocyclus $R^1$ aromatischer oder cycloaliphatischer Natur sein. Die heteroaromatischen Reste $R^1$ können 1 oder 2 der Heteroatome Sauerstoff, Stickstoff und/oder Schwefel enthalten.

Nach dem erfindungsgemäßen Verfahren können beispielsweise die Isoxazolcarbonsäureamide I, in denen der Rest $R^1$ für eine substituierte oder unsubstituierte Furyl-, Pyrrolyl-, Thienyl-, Pyridyl-, Pyrazinyl-, Pyrimidinyl-, Pyridazinyl-, Pyrazolyl-, Imidazolyl-, Oxazolyl-, Thiazolyl-, Isoxazolyl- oder Isothiazolyl-Gruppe steht, vorteilhaft hergestellt werden.

Ebenfalls vorteilhaft lassen sich nach dem erfindungsgemäßen Verfahren Isoxazolderivate I darstellen, in denen $R^1$ ein 5- bis 7-gliedriger Cycloaliphat ist, der ein oder zwei der Heteroatome Stickstoff und/oder bevorzugt Sauerstoff enthält. Als Beispiele für solche heterocycloaliphatischen Reste $R^1$ seien substituierte oder unsubstituierte Tetrahydrofuranyl-, Tetrahydrofuranyl-, 1,3-Dioxolanyl-, 1,3-Dioxanyl-, 1,4-Dioxanyl-, Oxepanyl-, 1,3-Dioxepanyl-, 1,4-Dioxepanyl-, 1,5-Dioxepanyl-, Pyrrolidinyl-, Imidazolidinyl-, Piperidinyl- oder Piperazinylgruppen genannt.

Weiterhin lassen sich nach dem erfindungsgemäßen Verfahren Isoxazol-4,5--dicarbonsäure-Diester II vorteilhaft zu den entsprechenden Isoxazol-5-carbonsäureamid-Derivaten umsetzen, in denen $R^1$ für eine substituierte oder unsubstituierte $C_6$- bis $C_{10}$-Aryl- oder $C_7$- bis $C_{12}$-Aralkylgruppe, insbesondere die Phenyl- oder die Benzylgruppe steht. Die Arylreste, insbesondere die Phenylgruppe, können dabei 1,2 oder 3 gleiche oder verschiedene $C_1$- bis $C_6$-Alkyl-, $C_1$- bis $C_6$-Alkoxy-, Halogen-, Nitro-oder Cyanogruppen als Substituenten tragen.

Die aliphatischen, araliphatischen oder cycloaliphatischen Reste $R^1$ der erfindungsgemäß herstellbaren Isoxazol-5-carbonsäureamide I können außerdem eine oder mehrere Doppelbindungen enthalten, wobei im Falle der cycloaliphatischen Reste $R^1$ diese Doppelbindungen sowohl endo- als auch exocyclisch sein können.

Die Art der Reste $R^2$ und $R^3$ der als Ausgangsverbindungen dienenden Isoxazol-dicarbonsäure-diester ist im allgemeinen für das erfindungsgemäße Verfahren nicht kritisch, es werden allerdings zweckmäßigerweise solche Reste $R^2$ und $R^3$, insbesondere Alkylreste, bevorzugt, welche den Ablauf der Umsetzung sterisch nicht behindern. Besonders bevorzugte Reste $R^2$ und $R^3$ sind $C_1$- bis $C_4$-Alkylgruppen. $R^2$ und $R^3$ können gleich oder verschieden sein, aufgrund der leichteren Verfügbarkeit werden aber Isoxazol-4,5-dicarbonsäure-diester, in denen diese Reste gleich sind, bevorzugt als Ausgangsverbindungen verwendet.

Das erfindungsgemäße Verfahren ermöglicht erstmals die einfache, hochselektive und wirtschaftliche Herstellung von Isoxazol-5-carbonsäureamid-Derivaten I, ausgehend von den Isoxazol-4,5-dicarbonsäure-diestern II.

## Beispiele

### Beispiel 1

50 g (0,25 Mol) 3-Methyl-isoxazol-4,5-dicarbonsäure-dimethylester wurden unter Stickstoff im Autoklaven zusammen mit 91,7 g (1,25 Mol) Diethylamin und 40 ml Methanol 8 h unter Eigendruck auf 60 °C erwärmt. Das Lösungsmittel und das überschüssige Amin wurden destillativ vom Rohprodukt abgetrennt, letzteres in Methyl-tert.-butylether (MTBE) aufgenommen, mit 1 n Salzsäure und anschließend mit Natriumcarbonatlösung gewaschen und 3-Methyl-4-methoxycarbonyl-isoxazol-5-N,N-diethylcarboxamid nach Trocknung über Natriumsulfat und destillativer Abtrennung des MTBE als Feststoff erhalten. Ausbeute: 76 %.

### Beispiel 2

50 g (0,25 Mol) 3-Methyl-isoxazol-4,5-dicarbonsäure-dimethylester wurden unter Stickstoff im Rührautoklaven zusammen mit 55 g (0,75 Mol) tert.-Butylamin und 100 ml Methanol 4 h unter Eigendruck auf 60 °C erwärmt. Nach der destillativen Abtrennung des Lösungsmittels und des überschüssigen Amins kristallisierte das Produkt 3-Methyl-4-methoxycarbonyl-isoxazol-5-tert.-butylcarbonsäureamid aus. Ausbeute: 94 %. Die weiteren Umsetzungen der Beispiele 3 bis 13 wurden analog Beispiel 1 durchgeführt. Die Reaktanten und Ergebnisse dieser Umsetzungen sowie die Daten der $^1$H-Kernresonanz(NMR)spektren der erhaltenen Verbindungen sind in der Tabelle aufgelistet.

In der Tabelle werden die folgenden Abkürzungen verwendet:
Me: Methyl; Et: Ethyl; i-Prop: Isopropyl; t-But: tert. Butyl; H: Wasserstoff; RT: Reaktionstemperatur; $CDCl_3$: Deuterochloroform; s: Singulett; d: Dublett; t: Triplett; q: Quadruplett; m: Multiplett.

Tabelle

| Beispiel | $R^1$ | $R^2$, $R^3$ | $R^4$, $R^5$ | Äquivalente $R^4R^5$-NH | RT | Ausbeute | 1H-NMR ($CDCl_3$) |
|---|---|---|---|---|---|---|---|
| 1 | Me | Me, Me | Et, Et | 5 | 60°C | 76 % | 1,1 (t, 3 H); 1,3 (t, 3 H); 2,5 (s, 3 H); 3,1 (q, 2 H); 3,6 (q, 2 H); 3,8 (s, 3 H). |
| 2 | Me | Me, Me | H, t-But | 3 | 60°C | 94 % | 1,5 (s, 9 H); 2,5 (s, 3 H); 4,0 (s, 3 H); 9,4 (s, 1 H). |
| 3 | Et | Me, Me | H, t-But | 3 | 70°C | 95 % | 1,3 (t, 3 H); 1,5 (s, 9 H); 2,9 (q, 2 H); 4,0 (s, 3 H); 9,3 (s, 1 H). |
| 4 | i-Prop | Me, Me | H, t-But | 3 | 70°C | 92 % | 1,3 (d, 6 H); 1,5 (s, 9 H); 3,4 (m, 1 H); 4,0 (s, 3 H), 9,0 (s, 1 H). |
| 5 | 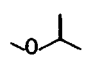 | Me, Me | H, t-But | 3 | 70°C | 68 % | 1,5 (s, 9 H); 1,6 (d, 3 H); 3,4 (s, 3 H); 4,0 (s,3 H); 4,8 (q, 1 H); 8,7 (s, 1 H). |
| 6 | 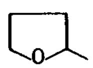 | Me, Me | H, t-But | 3 | 65°C | 73 % | 1,5 (s, 9 H); 1,9-2,4 (m, 4 H); 4,0 (s, 3 H); 3,8-4,1 (m, 2 H); 5,5 (dd, 1 H); 9,0 (s, 1 H). |

Forts. Tabelle

| Beispiel | $R^1$ | $R^2$, $R^3$ | $R^4$, $R^5$ | Äquivalente $R^4R^5$-NH | RT | Ausbeute | 1H-NMR ($CDCl_3$) |
|---|---|---|---|---|---|---|---|
| 7 | 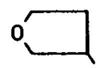 | Me, Me | H, t-But | 3 | 65°C | 85 % | 1,5 (s, 9 H); 2,2-2,5 (s, 2 H); 3,7-4,2 (m, 5 H); 4,0 (s, 3 H); 9,0 (s, 1 H). |
| 8 | 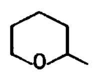 | Me, Me | H, t-But | 3 | 70°C | 86 % | 1,5 (s, 9 H); 1,5-2,1 (m, 6 H); 3,5-3,7 (m, 1 H); 4,0 (s, 9 H); 4,0-4,2 (m, 1 H); 4,8 (dd, 1 H); 8,8 (s, 1 H). |
| 9 | 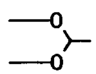 | Me, Me | H, t-But | 3 | 70°C | 70 % | 1,5 (s, 9 H); 3,4 (s, 6 H); 4,0 (s, 3 H); 5,8 (s, 1 H); 8,7 (s 1 H). |
| 10 | 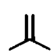 | Me, Me | H, t-But | 10 | 50°C | 95 % | 1,5 (s, 9 H); 2,1 (s, 3 H); 3,9 (s, 3 H); 5,4 (s, 1 H); 5,5 (s, 1 H); 8,3 (s, 1 H). |

Forts. Tabelle

| Beispiel | $R^1$ | $R^2$ , $R^3$ | $R^4$, $R^5$ | Äquivalente $R^4R^5$-NH | RT | Ausbeute | 1H-NMR ($CDCl_3$ ) |
|---|---|---|---|---|---|---|---|
| 11 | 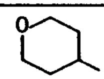 | Me, Me | H, t-But | 3 | 70°C | 61 % | 1,5 (s, 9 H); 1,6-2,1 (m, 4 H); 3,2-3,4 (m, 1 H); 3,5-3,6 (m, 2 H); 3,95 (s, 3 H); 4,0-4,1 (m, 2 H); 8,7 (s, 1 H). |
| 12 | 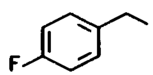 | Me, Me | H, t-But | 3 | 70°C | 75 % | 1,5 (s, 9 H); 3,8 (s, 3 H); 4,2 (s, 2 H); 6,8-7,2 (m, 4 H); 8,7 (s, 1 H). |
| 13 | 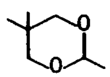 | Me, Me | H, t-But | 3 | 70°C | 68 % | 0,8 (s, 3 H); 1,4 (s, 3 H); 1,5 (s, 9 H); 3,6 (d, 2 H); 3,8 (d, 2 H); 3,95 (s, 3 H); 5,8 (s, 1 H); 8,5 (s, 1 H). |

Forts. Tabelle

| Beispiel | $R^1$ | $R^2$ , $R^3$ | $R^4$, $R^5$ |
|---|---|---|---|

Des weiteren sind die Verbindungen, welche unter den laufenden Nummern 14 bis 19 aufgelistet sind, nach dem erfindungsgemäßen Verfahren erhältlich.

| | | | |
|---|---|---|---|
| 14 | Me | Me, Me | H 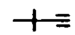 |
| 15 | Me | Me, Me | H 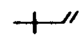 |
| 16 | Me | Me, Me | $R^4R^5$–NH = 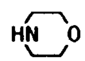 |
| 17 | Me | Me, Me | H 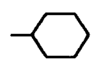 |
| 18 | Me | Me, Me | H 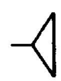 |
| 19 | Me | Me, Me | H 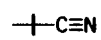 |

**Patentansprüche**

1. Verfahren zur Herstellung von Isoxazol-5-carbonsäureamid-Derivaten der allgemeinen Formel I

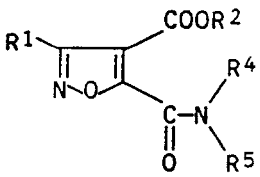

I

in der

$R^1$ ein aliphatischer Rest mit 1 bis 20, ein cycloaliphatischer Rest mit 3 bis 10, ein aromatischer Rest mit 6 bis 10, ein heteroaromatischer oder heterocyclischer Rest mit 3 bis 10 oder ein araliphatischer Rest mit 4 bis 12 Kohlenstoffatomen ist, wobei diese Reste mit unter den Reaktionsbedingungen inerten Substituenten substituiert sein können,

$R^2$ eine Alkylgruppe ist,

und in der die Reste

$R^4$ und $R^5$ gleich oder verschieden sind und für Wasserstoff, $C_1$- bis $C_6$-Alkyl-, $C_1$- bis $C_6$-Cyanoalkyl-, $C_2$- bis $C_6$-Alkenyl-, $C_2$- bis $C_6$-Alkinyl- und/oder für mit 1 bis 3 $C_1$- bis $C_4$-Alkyl-substituenten substituierte oder unsubstituierte $C_3$- bis $C_8$-Cycloalkylgruppen stehen

oder zu einem 5- bis 8-gliedrigen, cycloaliphatischen Ring, der ein weiteres Stickstoff- oder ein Sauerstoffatom enthalten kann, verbunden sind,

mit der Maßgabe, daß, falls $R^1$ die Methylgruppe und $R^4$ und $R^5$ eine Kombination aus Wasserstoff und der Isopropylgruppe darstellen, $R^2$ keine Ethylgruppe ist, dadurch gekennzeichnet, daß man Isoxazol-4,5-dicarbonsäure-diester der allgemeinen Formel II

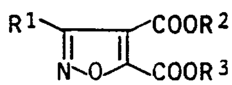

II

in der

$R^3$ für eine Alkylgruppe steht, die gleich oder verschieden von der Alkylgruppe $R^2$ ist, mit einem Amin der allgemeinen Formel III

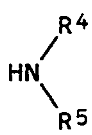

III

bei Temperaturen von 0 bis 100 °C umsetzt, wobei das Amin in mehr als äquimolaren Mengen, bezogen auf II, verwendet wird.

2. Verfahren nach Anspruch 1, dadurch gekennzeichnet, daß man die Umsetzung mit der bis 5-fach molaren Menge an Amin pro Mol Isoxazol-4,5-dicarbonsäure-diester durchführt.

3. Verfahren nach den Ansprüchen 1 und 2, dadurch gekennzeichnet, daß man 3-Methyl-, 3-Ethyl- oder 3-Isopropylisoxazol-4,5-dicarbonsäure-dimethylester mit der 3-fach molaren Menge an tert.-Butylamin umsetzt.

## Claims

1. A process for preparing isoxazole-5-carboxamide derivatives of the formula I

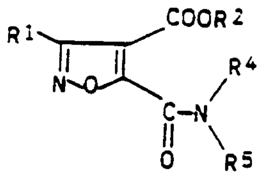

I

where

$R^1$ is an aliphatic radical of 1 to 20, a cycloaliphatic radical of 3 to 10, an aromatic radical of 6 to 10, a heteroaromatic or heterocyclic radical of 3 to 10 or an araliphatic radical of 4 to 12 carbon atoms, it being possible for these radicals to carry substituents which are inert under the reaction conditions,

$R^2$ is alkyl,

$R^4$ and $R^5$ are identical or different and are each hydrogen, $C_1$-$C_6$-alkyl, $C_1$-$C_6$-cyanoalkyl, $C_2$-$C_6$-alkenyl or $C_2$-$C_6$-alkynyl, and/or $C_3$-$C_8$-cycloalkyl which is unsubstituted or substituted by from 1 to 3 $C_1$-$C_4$-alkylgroups, or are linked to form a 5- to 8-membered cycloaliphatic ring which can contain another nitrogen or an oxygen,

with the proviso that $R^2$ is not ethyl if $R^1$ is methyl and $R^4$ and $R^5$ are a combination of hydrogen and isopropyl, which comprises reacting isoxazole-4,5-dicarboxylic diesters of the formula II

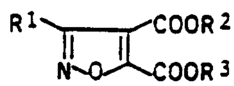

II

where

$R^3$ is alkyl which is identical to or different from $R^2$, with an amine of the formula III

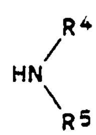

III

at from 0 to 100 °C, where the amine is used in more than an equimolar amount based on II.

2. A process as claimed in claim 1, wherein the reaction is carried out with up to 5 times the molar amount of amine per mole of isoxazole-4,5-dicarboxylic diester.

3. A process as claimed in claim 1 or 2, wherein the dimethyl ester of 3-methyl-, 3-ethyl- or 3-isopropylisoxazole-4, 5-dicarboxylic acid is reacted with 3 times the molar amount of tert-butylamine.

## Revendications

**1.** Procédé de préparation de dérivés d'amide d'acide isoxazol-5-carboxylique de la formule générale I

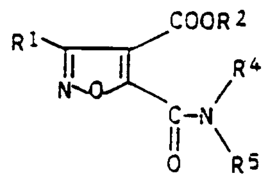

I

dans laquelle

$R^1$ est un reste aliphatique de 1 à 20 atomes de carbone, un reste cycloaliphatique de 3 à 10, un reste aromatique de 6 à 10, un reste hétéroaromatique, ou hétérocyclique de 3 à 10 ou un reste aliphatique de 4 à 12 atomes de carbone, ces restes pouvant être substitués avec des substituants inertes dans les conditions de la réaction

$R^2$ est un groupe alkyle et

dans laquelle les restes

$R^4$ et $R^5$ sont identiques ou différents et sont mis pour hydrogène, des groupes alkyle en C1 à C6, cyanoalkyle en C1 à C6, alcényle en C2 à C6, alkinyle en C2 à C6 et/ou cycloalkyle en C3 à C8 substitués ou non substitués avec 1 à 3 substituants alkyle en C1 à C4
ou sont liés en donnant un noyau cycloaliphatique de 5 à 8 chaînons, qui peut contenir un autre atome d'azote ou un atome d'oxygène

sous réserve que, lorsque $R^1$ représente le groupe méthyle et $R^4$ et $R^5$ une combinaison d'hydrogène et du groupe isopropyle, $R^2$ n'est pas un groupe éthyle, caractérisé par le fait qu'on fait réagir, à une température de 0 à 100 °C, un diester d'acide isoxazol-4,5-dicarboxylique de la formule générale II

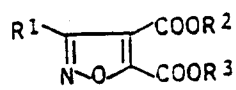

II

dans laquelle

$R^1$ est mis pour un groupe alkyle qui est identique à ou différent du groupe alkyle $R^2$ avec une amine de la formule générale III

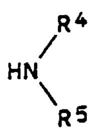

III

l'amine étant utilisée en plus d'une quantité équimolaire, rapportée à II.

**2.** Procédé selon la revendication 1, caractérisé par le fait que l'on effectue la réaction avec une quantité jusqu'à 5 fois molaire d'amine par mole de diester d'acide isoxazol-4,5-dicarboxylique.

**3.** Procédé selon les revendications 1 et 2, caractérisé par le fait que l'on fait réagir du diméthylester d'acide 3-méthyl-, 3-éthyl- ou 3-isopropylisoxazol-4,5 dicarboxylique avec une quantité 3 fois molaire de tert-butylamine.